(19) Europäisches Patentamt
European Patent Office
Office européen des brevets

(11) Veröffentlichungsnummer: **0 223 850**
**B1**

(12) **EUROPÄISCHE PATENTSCHRIFT**

(45) Veröffentlichungstag der Patentschrift: **11.04.90**

(51) Int. Cl.$^5$: **C 07 D 207/16**, A 61 K 31/40

(21) Anmeldenummer: **86904120.2**

(22) Anmeldetag: **20.05.86**

(88) Internationale Anmeldenummer:
**PCT/EP86/00304**

(87) Internationale Veröffentlichungsnummer:
**WO 86/07053 04.12.86 Gazette 86/26**

(54) **ARZNEIMITTEL, DIE DERIVATE DES PROLIN ODER HYDROXYPROLIN ENTHALTEN.**

(30) Priorität: **20.05.85 DE 3518078**

(43) Veröffentlichungstag der Anmeldung:
**03.06.87 Patentblatt 87/23**

(45) Bekanntmachung des Hinweises auf die Patenterteilung:
**11.04.90 Patentblatt 90/15**

(84) Benannte Vertragsstaaten:
**CH DE FR GB IT LI NL**

(56) Entgegenhaltungen:
**DE-B-1 098 514**
**US-A-3 282 917**
**US-A-3 891 765**

**Chemical Abstracts, vol. 102, no. 1, 7 January 1985, Columbus, Ohio, US, see page 55, abstract no. 572n**

**Die Akte enthält technische Angaben, die nach dem Eingang der Anmeldung eingereicht wurden und die nicht in dieser Patentschrift enthalten sind.**

(73) Patentinhaber: **Hoerrmann, Wilhelm, Dr.**
**Staltacherstrasse 34**
**D-8127 Iffeldorf (DE)**

(72) Erfinder: **Hoerrmann, Wilhelm, Dr.**
**Staltacherstrasse 34**
**D-8127 Iffeldorf (DE)**

(56) Entgegenhaltungen
**Chemical Abstracts, vol. 101, 1984, Columbus, Ohio, US R.P.A. Inigo et al.:"Terpenoids and trans-4-hydroxy-N-methyl-L-proline from Erythroxylon argentinum", see page 401, abstract no. 126897v**

**Chemical Abstracts, vol. 87, 1977, Columbus, Ohio, US A.A. Kurganov et al.:"Stability of copper(II) complexes with N-Alkyl-alpha-amino acids", see page 451, abstract no. 29872t**

**Chemical Abstracts, vol. 100, 1984, Columbus, Ohio, US S.Scuito et al.:"The identification of 4-hydroxy-N-methyproline in the red alga Chondria coerulescensspectral information", see page 356, abstract no. 99883h**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 223 850 B1

(56) Entgegenhaltungen
Chemical Abstracts Service, Registry Handbook 1978 Supplement, "L-Proline, 4-hydroxy-1-methyl-,cis-C6H11N03", see left column, No. 67463-44-9

Chemical Abstracts, vol. 63, no.6, 13 September 1965, Columbus, Ohio, US Tadashi Suyama et al.:" N-Alkylamino acids.V." see column 7095-h

**Beschreichung**

Die Erfindung betrifft ein Arzneimittel, das als Wirksubstans mindestens ein N-Alkyl-Derivat des Prolin oder Hydroxyprolin, insbesonders das N-Methyl-Derivat des Prolin oder Hydroxyprolin oder dessen pharmazeutisch annehmbaren Abkömmling, ggf. zusammen mit üblichen Trägern und/oder Hilfsmitteln, enthält. Dabei ist zu beachten, daß das Derivat des Prolin oder Hydroxyprolin in der L oder D Konfiguration vorliegen kann, daß es sich bei dem Derivat des Hydroxyprolin um das cis- oder trans-Isomere handeln kann, und daß sich die Hydroxylgruppe des Derivates des Hydroxyprolin an verschiedenen C Atomen des Ringes von C3 bis C5 befinden kann.

Die Wirkung der Arzneimittel kann durch die Zugabe von N-Methyl-glycin gesteigert werden. Dieser Substans kommt auch eine Eigenwirkung zu. (N-Methyl-glycin wird in der Literatur auch als Sarkosin bezeichnet.)

Die JP—A—164 718 (1984), referiert in Chemical Abstracts 102 (1) (1985) 572n, beschreibt die Verwendung von L-Prolin zur Hemmung der carcinogenen Aktivität von N-Nitrosoverbindungen.

Strukturformeln der aufgeführten Verbindungen:

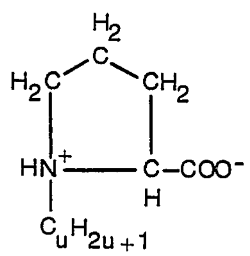

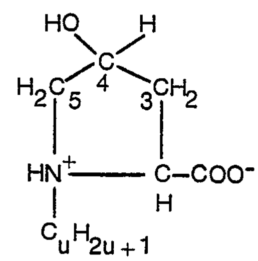

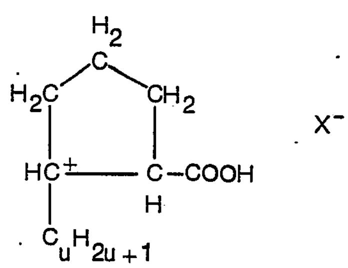

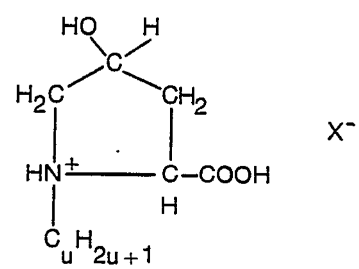

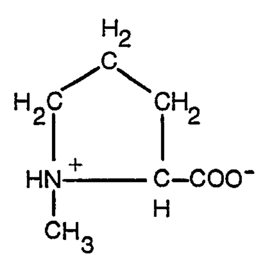

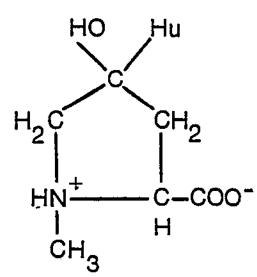

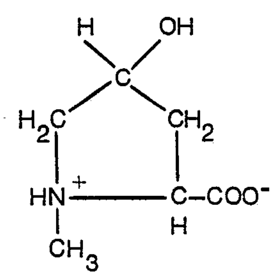

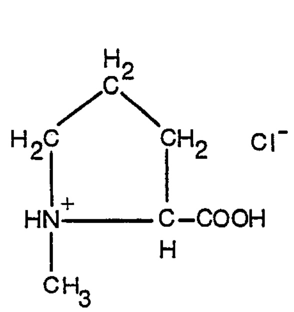

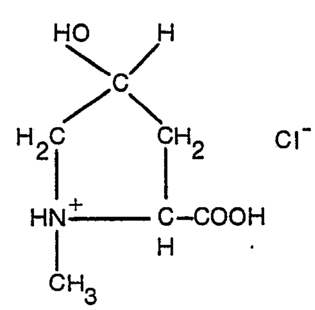

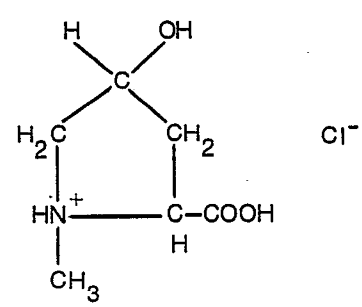

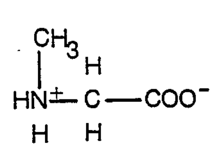

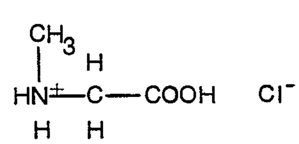

Alle Verbindungen können nach an sich bekannten Verfahren hergestellt werden.

Beispielhaft wird dies für cis- und trans- N-Methyl-4-hydroxy-L-prolin und ihre Hydrochloride erläutert: Man geht vom cis oder trans-4-hydroxy-L-prolin aus, die beide bekannte Substanzen und im Handel erhältlich sind. Hierauf wird in Wasser mit 1.17 Aeq Formaldehydlösung (37%ig) reduktiv mehyliert durch Hydrieren in Gegenwart von 5% Pd auf Kohle bei Raumtemperatur bis zum Stillstand der $H_2$-Aufnahme (45—60 Min.). Das durch Eindampfen isolierte Rohprodukt wird noch durch Umkristallisieren aus Ethanol/Wasser 8:2 gereinigt.

Analysendaten von N-Methyl-cis-4-hydroxyl-L-prolin:
- Aspekt: Praktisch farblose, nadelförmige Kristalle.
- Zersetzungspunkt: Bei ca. 180°C (nicht exakt reproduzierbar)
- Gehalt: 99,6% ($HCl O_4$)

Analysendaten von N-Methyl-trans-4-hydroxy-L-prolin:
- Aspekt: Praktisch farblose, nadelförmige Kristalle.
- Zersetzungspunkt: 239,5 bis 241°C.
- Gehalt: 104% ($HCl O_4$)

Die Hydrochloride der obigen Verbindungen werden durch Lösen der N-Methyl-Verbindungen in aequivalenter Menge verdünnter Salzsäure und Eindampfen zur Trockene erhalten.

Analysendaten von N-Methyl-4-hydroxy-L-prolin-Hydrochlorid:
- Aspekt: Praktisch farbloses Pulver.
- Schmelzpunkt: 177 bis 179°C.
- Gehalt: 100,1% ($Ag No_3$)

Analysendaten von N-Methyl-trans-4-hydroxy-L-prolin-Hydrochloride
- Aspekt: Praktisch farbloses Pulver.
- Schmelzpunkt: 181 bis 183°C zers.
- Gehalt: 99,6% ($Ag NO_3$)

Die Ergebnisse, die bisher bei Krebs und Viruserkrankungen, bei Erkrankungen des Gefäß- und Nervensystems medikamentös therapeutisch erzielt werden können, lassen vielfäch in hohem Maße zu wünschen übrig, sei es, daß die Wirkungen zu gering, die Nebenwirkungen aber zu hoch sind. Der Erfindung liegt daher die Aufgabe zugrunde, ein Arzneimittel zur Verfügung zu stellen, das bei weniger Nebenwirkungen, die genannten krankhaften Veränderungen in einem besseren Maße lindern oder heilen kann. Diese Aufgabe wird durch ein Arzneimittel dieser Art gelöst.

Die erfindungsgemäßen Arzneimittel können die Derivate des Prolin und Hydroxyprolin auch in Form von pharmazeutisch verträglichen Abkömmlingen enthalten, besonders solche, die im Körper in die freie Form der Derivate übergehen. Dazu gehören insbesondere Alkalisalze, Erdalkalisalze, Säureadditionssalze, Ester, Amide und Ether von Prolin bzw. Hydroxyprolin und deren N-Alkyl-derivaten sowie Oligo- und Polypeptide davon.

Die erfindungsgemäßen Arzneimittel werden in grundsätzlich gleicher Weise verabreicht wie die gewöhnlichen Aminosäuren, also bevorzugt peroral oder intravenös, bzw. zentralintravenös. Auch die Zufuhr in Tabletten, Dragees, Injektions- und Infusionslösungen ist die gleiche.

Auch die Dosierung liegt im üblichen Aminosäurenbereich, wobei man 0,01—0,1 g/kg pro Tag zugrunde legen kann.

Die für alle Aminosäuren geltenden Kontraindikationen sind auch hier zu beachten.

Die pharmazeutische Herstellung und Verarbeitung erfolgt in allgemein üblicher Weise und berücksichtigt die gesetzlichen Bestimmungen in Hinsicht auf Substanzreinheit, Sterilität, Pyrogenfreiheit u.s.w.

Als Beispiel für die ausgezeichnete Wirkung der erfindungsgemäßen Substanzen auf Krebszellen sind die nachfolgenden Untersuchungen as Zellkulturen aufgeführt: Bei diesem "stem cell assay" eines Astrocytoms menschlicher Herkunft wird die Zahl der Kolonien, die der Tumor bildet, quantitativ erfaßt. Je niedriger also die Zahl, desto stärker die Wirkung. Im vorliegenden Versuch wurde Sarkosin als freie Base, sowie N-Methyl-cis-4-hydroxyl-L-prolin und N-Methyl-trans-4-hydroxy-L-prolin, beide als Hydrochlorid, verwendet. Die Gewichtsangaben sind Mikrogramme.

| | |
|---|---|
| Kontrolle ohne Zusatz. | 814 |
| Kontrolle 10 Sark | 598 |
| 100 Methyl trans + 10 Sark | 285 |
| 10 Methyl trans + 1 Sark | 450 |
| 1 Methyl trans + 0,1 Sark | 544 |
| 100 Methyl cis + 10 Sark | 300 |
| 10 Methyl cis + 1 Sark | 320 |
| 1 Methyl cis + 0,1 Sark | 280 |

Ergebnis:

Während bereits der Zusatz von Sarkosin zu einer Reduzierung der Kolonienbildung führt, wird dieser Effekt wesentlich verstärkt durch die Zugabe der N-Methyl-Derivate des Hydroxyprolins. Als ganz besonders bemerkenswert ist hervorzuheben, daß das cis Isomere seinen tumorhemmenden Effekt selbst noch dann voll beibehält, wenn die Wirkstoffmenge auf ein Hundertstel reduziert wird.

**Patentansprüche**

1. Arzneimittel, enthaltend als Wirksubstanz mindestens ein N-Alkylderivat des Prolin oder Hydroxyprolin oder dessen pharmazeutisch annehmbaren Abkömmling, ggf. zusammen mit üblichen Trägern und/oder Hilfsmitteln.

2. Arzneimittel nach Anspruch 1 wobei es sich um die L oder D Konfiguration der Derivate des Prolin oder Hydroxyprolin handelt.

3. Arzneimittel nach einem der Ansprüche 1—2, wobei es sich um das cis oder trans Isomere der Derivate des Hydroxyprolin handelt.

4. Arzneimittel nach einem der Ansprüche 1—3, wobei sich die Hydroxylgruppe der Derivate des Hydroxyprolins an verschiedenen C Atomen des Ringes von C 3 bis C 5 befindet.

5. Arzneimittel nach einem der Ansprüche 1—4, dadurch gekennzeichnet, daß es sich um die N-Methyl-Derivate des Prolin oder Hydroxyprolin handelt.

6. Unterstützung der Wirkung eines Arzneimittels nach den Ansprüchen 1—5, durch die Zufügung von N-Methyl-glycin.

7. Arzneimittel, bzw. Substanzen nach einem der Ansprüche 1—6 dadurch gekennzeichnet, dass die Aminosäuren im Form ihrer Säureadditionssalze vorliegen.

**Revendications**

1. Médicament contenant, en tant que matière active, au moins l'un des dérivés N- d'alkyle de la proline ou de l'hydroxyproline, ou bien son dérivé pharmaceutiquement acceptable, le cas échéant conjointement avec des porteurs et/ou adjuvants usuels.

2. Médicament d'après la revendication 1, les dérivés de la proline ou de l'hydroxyproline étant présents dans leur configuration L- ou D-.

3. Médicament d'après l'une des revendications 1—2, les dérivés de l'hydroxyproline étant présents sous forme d'isomers de type cis ou trans.

4. Médicament d'après l'une des revendications 1—3, le groupe hydroxyle des dérivés d'hydroxyproline rattaché à ceux des atomes C de l'anneau qui se trouvent dans les positions C3 à C5.

5. Médicament d'après l'une des revendications 1—4, caractérisé en ce qu'il s'agit de dérivés N-méthyliques de la proline ou de l'hydroxyproline.

6. Perfectionnement de l'action d'un médicament d'après les revendications 1—5, en y rajoutant de la glycine méthylique de type N-.

7. Médicaments ou substances d'après l'une des revendications 1—6, caractérisés en ce que les aminoacides sont présentes sous forme de sels d'addition acidique.

**Claims**

1. Drug containing as its active substance at least one N-alkyl derivative of proline or hydroxyproline, or of its pharmaceutically acceptable derivative, if necessary together with usual carriers and/or adjuvants.

2. Drug according to claim 1, being the L or D configuration of said proline or hydroxyproline derivatives.

3. Drug according to any of claims 1—2, being a cis or trans isomer of said derivatives of hydroxyproline.

4. Drug according to any of claims 1—3, the hydroxyl group of the derivatives of hydroxyproline being attached to various C atoms of the ring, in positions C 3 through C 5.

5. Drug according to any of claims 1—4, characterized in that it is a N-methyl derivative of proline or hydroxyproline.

6. Enhancement of the efficiency of any drug according to claims 1—5, by adding N-methyl glycine.

7. Drugs or substances according to any of claims 1—6, characterized in that said aminoacids are present as acid addition salts.